# EUROPEAN PATENT APPLICATION

(11) **EP 0 859 235 A1**
(43) Date of publication of application: **19.08.1998**
(21) Application number: 97430006.3
(22) Date of filing: 14.02.1997
(51) Int. Cl.: G01N 33/28, G01N 1/00, B01D 29/66

(54) **Analytical system**

(71) Applicant: BP CHEMICALS S.N.C., 92400 Courbevoie (FR); BP FRANCE S.A., 95866 Cergy Pontoise Cedex (FR)
(72) Inventor: The designation of the inventor has not yet been filed
(74) Representative: Lassalle, Pierre-Dominique

(57) **Abstract**

An analytical system for sampling a feed in a process line e.g. crude oil has first and second lines from the process line to first and second separation means, lines from said separation means to an analytical means e.g. an NIR spectrophotometer and computer, and means for cleaning the separation means. In the method, the flow of oil sample flows in one direction from process line to first separation means and analytical means at the same time as a backwash liquid passes form the back of the second separation means towards the process line to clean it.

## Description

The present invention concerns an analytical system, in particular one for liquids which contain insoluble solids or on cooling or ageing can contain deposits, which cause fouling of analytical equipment.

Increasing numbers of industrial processes with liquid flowing in a pipe are sampled on or at line and analysed to measure a property of the liquid, and then the process controlled by adjustment of its conditions to keep the property at the desired value. Among such processes are hydrocarbon conversion, separation and blending processes in refineries. Such hydrocarbons often contain solids which are in suspension, or in solution and capable of separation on cooling. After a period of use the analytical equipment becomes fouled with the actual or deposited solids. To reduce this the liquid can be filtered, after cooling if required, to remove solids, and then the filtrate analysed. However, the porosity of filter soon becomes reduced as the amount of solid removed on tile filter increases. Eventually the rate of filtration is too slow to be useful commercially. The analysis is then stopped while the filter is removed cleaned and replaced. During this period the analysis cannot be performed, hence the process is not controlled, so deviations from desired behaviour can occur.

A process has been discovered enabling continuous analysis to be performed and hence continuous control.
The present invention provides a method of on line analysis of a feed comprising a liquid and containing at least one material capable of interfering with the analytical apparatus, which method comprises the steps
(i) passing a first sample of said feed containing said material at least in part as a separate phase to a first separation means to give a first fluid and a first residue retained by said first separation means,
(ii) analysing said first fluid
(iii) removing said first residue from said first separation means
(iv) passing a second sample of said feed containing said material at least in part in a separate phase to a second separation means to give a second fluid and a second residue retained by said second separation means,
(v) analysing said second fluid
(vi) removing said second residue from said second separation means,
   wherein steps (iii) and (v) are performed at least to some extent at the same time.

The feed will be described with reference to a hydrocarbon liquid, but may be any liquid, organic or inorganic, which contains insoluble solids e.g. sand, by product salts, or which contains dissolved solids, which solids can be become insoluble or cooling e.g. dissolved salts alternatively or in addition the liquid may contain a separate liquid contaminant phase e.g. as droplets. In particular the organic liquid is a hydrocarbon liquid e.g. an oil, especially one that is or is derived from a crude oil. Crude oil can contain insoluble solids e.g. sand, drilling solids, salts, formation rock, dust, sediments, as well as organic solids at least partly in or out of solution, such as asphaltenes and waxes and/or water. Preferably the crude oil has been substantially desalted and dewatered before sampling but is often at higher temperature up to 150°C and higher pressure e.g. 1-10 bar. The presence of soluble waxes is particularly important as crude oil at the well head is often at up to 150°C e.g. 50-150°C such as 60-100°C e.g. 60-80°C and on cooling in plant lines e.g. to 20-40°C can often deposit waxes; such waxes could deposit on analytical equipment. Such cooling may also cause dissolved water in the hot oil to form a cloud of small droplets. The oil may also be a residue from a distillation, whether at atmospheric or reduced pressure, of crude oil, in which case it often contains the above organic solids. The oil may be a feed to a cracker, e.g. a catalytic or thermal cracker, especially one with a fluidized bed; examples of such feeds are vacuum distillation products or residues Tie oil may also be a residual oil, a distillate fraction of atmospheric boiling range, which is used as a source for lubricating oils, but often has a high wax content; dewaxing of the fraction gives bright stock, a base oil for lube oils. The oil may also be a gas oil or diesel oil, which often contains waxes, or may be a gasoline fraction which often contains unsaturated materials, which are resins or gums, or which form these or standing and/ageing. The solids which cause the fouling problems in the analytical equipment may be crystalline, waxy or resinous.

Each of the first and/or second residue removed may be a solid or insoluble liquid, or both may be removed. The liquid may be removed e.g. water from a liquid hydrocarbon in a liquid separator e.g. cyclone or settling tank, especially in the case of a separate continuous phase. Prior to this separation or instead of this separation, the liquid, present as droplets, may be contacted with a surface in a coalescer to retain the droplets and preferably to allow or encourage them to form into large droplets which settle out into a separate continuous liquid phase, which may be removed periodically, continuously or continually. The coalescers may comprise bodies of mats or beds or layers of porous or fibrous solids. The mats/beds/layers may be in the same apparatus as a liquid separator or prior to the liquid separator. Thus each of the first and second separating means, may comprise means for separating liquid especially as droplets, e.g. a coalescer and/or cyclone.

The degree of cooling and hence the temperature of the first and second heat exchangers (and hence the subsequent first and second separation means) may be such as to form insoluble solids or liquids or both. The temperature of those heat exchangers may be 5-70°C e.g. 10-50 especially 10-20°.

Preferably, at least one of the first and second residues, especially both, is a solid. Each of the first and second solid separation means which may be the same or different is usually a filter but may also be a gravity concentrator e.g. centrifuge or cyclone. The separator may be capable of retaining solids of greater than 500 microns in diameter. The separator is preferably a filter of average porosity less than 300 microns especially less than 150 microns, such as less than 100 microns e.g. 5-200 especially 5-50 or 50-120 microns. The filter is preferably an inorganic filter e.g. of ceramic glass, or metal fibres or filaments in a bed, but may be organic e.g. cellulosic such as a paper or polymeric e.g. polyamide or polyolefin fibres or filaments. The solid or liquid separation means removes insoluble solids or liquids to leave a first or second fluid which is the analysate for the analysis. The liquid passed for analysis are thus usually at a temperature not more than that of the analysis and advantageously 1-5°C below that temperature to ensure no deposition; thus if required the liquid may be heated via an exchanger between the separation means and the analyser. The analysate on entry to the analyser may be at 0-90°C, but especially 20-40°C or 40-70°C.

The first and second fluids are the analysates, and may be produced in amounts of 1-5ml per analysis in the case of discontinuous i.e. continual analysis or continuously in the case of continuous analysis.

The analysis may be chromatographic, e.g. gas phase or high liquid phase chromatographic, in which the sample of fluid is injected into a column; the process of the invention removes the solids which might otherwise cause fouling on the inlet to the column. Preferably the analysis is spectroscopic e.g. UV, visible 200-800nm, infra red (e.g. 2600nm to 10,000nm) but preferably is near infra red e.g. at 600-2600nm especially 1200-2600 such as 1600-2600 or 2000-2500nm. The spectroscopic analysis is in a standard cell kept at a fixed temperature through which the appropriate radiation passes, the path length in the cell varying with the wavelength e.g. from 0.1-1mm for IR, 0.5-10mm for NIR, 1-5cm for visible and 5-10cm for UV radiation. Advantageously the analysis by NIR is by mathematical e.g. statistical treatment of the absorbance data such as partial least squares treatment to determine a calibration model and Regression Equation to determine the property or analysis required.

The residue retained by the first and second separation means may be an insoluble liquid but is preferably a solid optionally with such a liquid. The residue may also contain some of the first or second fluids respectively either adherent thereto e.g. in the case of a filter cake, or in suspension therein in the case of a gravity concentrator. The residue may be removed from the first or second separating means e.g. filter or coalescer by contact with a first or second liquid respectively. The liquid may be passed over the separation means to wash away the residue from its surfaces but preferably is passed backwards through the separation means e.g. filter to remove any residue in its pores as well; the passage may be under substantially atmospheric or under super atmospheric pressure e.g. 1-10 bar. Each liquid may be a solvent or non solvent for at least some of the residue, and especially in the case of organic solids e.g. ones deposited on cooling the liquid e.g. oil may be at a temperature at least that of the previous separation step and preferably at least 5°C higher e.g. 5-60°C higher. In the case of organic solids deposited from liquid hydrocarbons such as crude oil and fractions thereof e.g. waxy lube precursor fractions aid residue oils, the liquid may be itself a hydrocarbon e.g. gas oil or another petroleum oil cut especially one rich in aromatic hydrocarbons, or indeed a liquid aromatic hydrocarbon, or clean crude; indeed the liquid may be some of the fluid separated in a previous separation step e.g. filtrate, which may be from the same or a different separation step. When the residue is an insoluble liquid, the first or second liquid may physically wash it off the separating means e.g. coalescer, or may dissolve out the insoluble liquid at the contact temperature. The same liquid as mentioned above may be used.

The effluent mixture of retained residue and first or second liquid obtained in the backwash step may be discarded as a concentrated waste product and used e.g. as fuel, or may be returned to the stream from which the original first and second samples were taken, but ideally downstream thereof.

Periodically, the separation means may be rinsed with a strong solvent for organic insolubles e.g. a halo hydrocarbon such as carbon tetrachloride.

In many cases the fluids in the process line contain both solids insoluble at the time of sampling and also dissolved solids which separate on cooling e.g. to below the cloud point. The stream being sampled may be at a temperature of 25-160°C e.g. 60-100°C. If the analytical apparatus is at a lower temperature than that of the stream being sampled, then it is preferred to cool the sample before the separation step. The sample may be cooled and all the solids removed at once, or the sample may be submitted to a separation step to remove insolubles, and then the fluid obtained cooled to deposit more insolubles, which are then in turn separated prior to analysis; if desired this process of stepwise cooling and separation may be repeated more than once. If desired, the sample may be submitted to a series of separation steps which remove insolubles of ever smaller size e.g. filters of progressively smaller pore size. Thus the first insolubles may be removed in a coarse filter, and then the second (and subsequent if any) insoluble removed on finer filters; this approach may be used with a stepwise cooling as well if desired. The filters may also be of substantially the same pore size if desired. The order of the filters may also be the reverse, a small pore size first e.g. as described above, especially for removing solids insoluble in the sample and a larger size e.g. up to 300 microns such as 50-300 or 100-200 microns subsequently especially for removing solids which had formed on cooling. In a preferred embodiment a sample of the feed is removed from its line and passed to a third solid separation means to produce a third fluid and leave a third residue, said third fluid is cooled in a first heat exchanger to produce insoluble material, which is separated in the first separation means to give the first fluid which is then analyzed; if desired the cooling and removal of insoluble material may be repeated at least once more e.g. via fourth solid separation means, prior to the first solid separation means. Similarly between removal of the second sample from the line and the second solid separation means, there may be fifth solid separation means to produce fifth fluid, and cooling in a second heat exchanger to form insoluble material prior to separation in the second solid separation means; again the cooling and separation may be repeated at least once more via sixth solid separation means. The cooling may reduce the temperature of the material passing to the first or second separation means to 20-150°C especially 20-90°C or 20-40°C.

The act of cooling before the first and second separation steps may result in the formation of the insoluble solids e.g. waxes as above and/or the separated liquid e.g. fine droplets such as of water. The first/second separators means may then comprise the solid or liquid separating means, while upstream of each may be the third (and subsequent) and/or fifth and subsequent separating means. Thus insolubles in the sample may be removed in the third separator, the third fluid may be cooled to below the cloud point producing insoluble solids and/or liquids, and the insolubles separated in the first separator; the same approach would apply also with the fifth and second separators. Thus the oil sample may pass through a seventh separating means to remove insoluble solids, a heat exchanger to cool it partly to condense water but not wax, a third separating means which is a coalescer to remove water, a first heat exchanger to cool the sample further to form solid wax and a first separator to remove the solid wax. Analogous apparatus would be used for passage of the sample through the third separator to the second separator. If desired the initial heat exchanger may cool the fluid to condense water and wax, the coalescer can remove the water, and then the separated wax can be melted by heating the line to the analysis all or passing the product through a heat exchanger at a higher temperature than the coalesce; by this means the amount of water removed is increased for a given analysis temperature.

In some cases, especially with deposits of resins and gums, e.g. from gasoline or crude oil, the rate of deposition is slow compared to the rate of solid separation, so that it may be desirable to stand the sample for a requisite time to complete any deposition before the separation step. Under these circumstances there may be insufficient time to allow backwashing of one filter during active use of the other filter. Then a third sample is taken and the separation (and optional cooling step) and analysis are performed analogous to those with the first and second sample, while one of the separation means is being backwashed. Further sampling and backwash may also be performed if desired, in a corresponding way to the first and second operations.

In the case of the first and second samples (and third if present), a separate piece of analytical equipment may be used for each sample, but conveniently the same piece is used e.g. the same NIR cell, with alternate feeds thereto of the first and second fluids (and third) if present via a first multiway valve. In this specification a multi way valve has at least 3 ways through it.

Similarly the effluent from the backwash step (iii) and (vi) from each of the separation means (whether with stepwise removal of insolubles or not) may be passed separately to waste or returned to the main line. Preferably the effluent from at least 2 separation means and preferably all those after a cooling step are combined; the effluents from the separation means removing residue insoluble in the line e.g. inorganic material such as sand and rock may be removed separately from the rest, or combined with the rest, via a second multi way valve.

The first and second fluids which are passed into or trough the analytical equipment may be discarded e.g. used as fuel or returned to the main line, or may be used as backwash liquid. Thus the first fluid after analysis may be passed as backwash liquid to or through the second separation means, and when the second means is clean and/or the first means becomes of reduced transmission, the second fluid may be passed as backwash to the first separation means etc. Conveniently with a single piece of analytical equipment for both fluids, the flow from the feed as the first or second sample can be coordinated with flow of first or second fluid as backwash for the second or first separation means (and earlier separation means if separation is performed stepwise).

Preferably however the backwash liquid is different from the liquid being sampled, especially being of lower boiling point, so that for example gasoline can be used for backwashing deposits from gas oil, and gas oil used for backwashing deposits from crude oil. The backwash liquid can be passed to each separating means individually and the effluent removed separately or combined e.g. via the second multi way valve. Preferably however the first backwash liquid passes to the first separation means and then the effluent produced passes through any heat exchanger and/or other separation means in the line from the process line to the first separation means; in this way any deposits of solid and/or liquid are progressively stripped from the entire line. The effluent from the nearest exchanger/separation means to the process line can then be diverted from the first line before it reaches the process line e.g. by a third multi way valve. The same approach applies with the stripping with the second backwash liquid.

Conveniently therefore there are 2 circuits with the first sample passing via, in order, optionally the third multiway valve, third separation means if present, first heat exchanger cooler if present, first separation means to the analytical apparatus, while the second backwash fluid is being passed via the second separation means, second heat exchanger if present, fifth separation means if present and then diverted to the effluent disposal via a fourth multiway valve. This operation alternates with the reverse, with second fluid passing through in the reverse direction to the above backwash liquid and through to the analytical equipment, and the first backwash liquid passes in the reverse direction to the first fluid.

All the liquid part of the first fluid sample may, after the first separation step, pass into the analytical apparatus, but since the rate and ease of separation depends on the particle size of the solids aid/or droplet size of insoluble liquids, and it is often desired to remove very small particles and/or droplets, the volume of liquid taken from the process line would inevitably be small. It is preferred to have a larger flow rate for the first separation means than for the analytical apparatus, so preferably only a portion of the first fluid from the first separation means is passed for analysis (e.g. 5-30% e.g. 10-20% by volume), while the remainder is recycled to effluent or returned to the process line. When there is a preliminary third separation means and/or first heat exchanger, the flow rate to it/them is preferably higher still so preferably only a portion of the third fluid from the third separation means, is passed to the first exchanger (if present) and first separation means (e.g. 1-20% e.g. 2-10% by volume based on the fluid passed to the third separation means), while the remainder is recycled to effluent or returned to the process line. The percentage of the flow rate of the first fluid passed to the analytical apparatus to the fluid passed to the third separating means may be 0.1-10% e.g. 0.2-5% by volume. The same approach applies with the second sample.

Thus there may be 2 separate highest flow rate circuits (one for the sample and one for the backwash) and 2 separate medium flow rate circuits (one for the sample and one for the backwash) and one or two slow flow rate circuits for the analysate; preferably however apart from the passage through the specific third/first separation means and first exchanger, and second/fifth separation means and second exchanger, the rest of each pair of circuits are where ever possible communal.

The present invention also provides apparatus for sampling a feed in a process line and analyzing it, which apparatus provides a first and second line for attachment to said process line (preferably either directly or indirectly via a multi way valve), a first and second separation means joined to said process line by said first and second line, a first Analytical Means joined to said first separation means by a third line, and said first Analytical Means or a second Analytical Means being joined to said second separation means by a fourth line, and means for cleaning said first and second separation means with first and second liquids.

Preferably, between the process line and the first and second separators, the first and second lines respectively have fifth and sixth valves, adapted to be operated so that at all times during analysis at least one is open to movement of fluid to the analytical equipment and preferably the valves are open and closed alternately. In particular the fifth and sixth valves are combined as a seventh multi way valve joined to the process line.

Advantageously between the process line and the first separation means is one or more separation means e.g. third separation means and/or one or more heat exchangers e.g. a first heat exchanger to cool fluid in the first line and similarly between the process line and the second separation means is one or more separation means e.g. fifth separation means and/or one or more heat exchangers e.g. a second heat exchanger to cool fluid in the second line.

The first and second Analytical Means may be the same in which case it is joined by third and fourth lines to said first and second separation means respectively. Advantageously in this case, but not limited thereto, the means for removing residue from the first and second separating means has separate first and second input lines thereto but output lines for effluent that meet.

At least one of the first and second Analytical Means, and preferably both, has an exit line for analysate which is joined to at least one of the second and first input lines respectively to the second and first Separation means, to allow excess analysate from one separator to backwash the other separator. When both Analytical Means are the same, the exit line for analysate may diverge into 2 lines leading to the third and fourth lines at flow diverters e.g. multiway valves, allowing alternate passage of liquid down third line through Analytical Means, exit line and back to fourth line and second separation means, and corresponding passage of liquid down fourth line to exit line and back to the third line.

When only a portion of first fluid passes to the analytical apparatus, the rest being recycled, the apparatus can have in order from the process line a 3 way valve, optional third separation means, optional first heat exchanger, first separation means, optional first multiway valve, and analytical apparatus with an analysis recycle line leading to effluent, as well as direct recycle lines from the first separation means leading preferably via an eighth multi way valve to effluent. The apparatus has a similar arrangement from the process line to the second separation means and analytical apparatus to effluent preferably via the eighth multi way valve. Preferably the apparatus has a line from the seventh multi way valve to the third separation means in parallel to a line from said valve to fifth separation means, the third separation means being joined to the effluent line especially via a ninth multi way valve and/or a third heat exchanger, and the fifth separation means being joined to the effluent line, especially via a ninth multi way valve and/or a third or fourth exchanger, and also being joined to a second heat exchanger and hence to the second separation means and to the analytical apparatus. The third/fourth heat exchanger in the recycle line is preferably linked to preheat the incoming backwash liquid prior to joining first or the second separation means, or both especially via a tenth multi way valve.

The invention is illustrated in and described with respect to the accompanying drawings, which are flow diagrams in which,
Figs. 1 and 2 show the apparatus with direction of flows through the apparatus at alternate times.
Figs 3 and 4 show a modified apparatus with the direction of flows throughout it at alternate times.

In the Figures, pressure valves, flow meters, pressure indicators and similar ancillary equipment have been omitted on grounds of clarity.

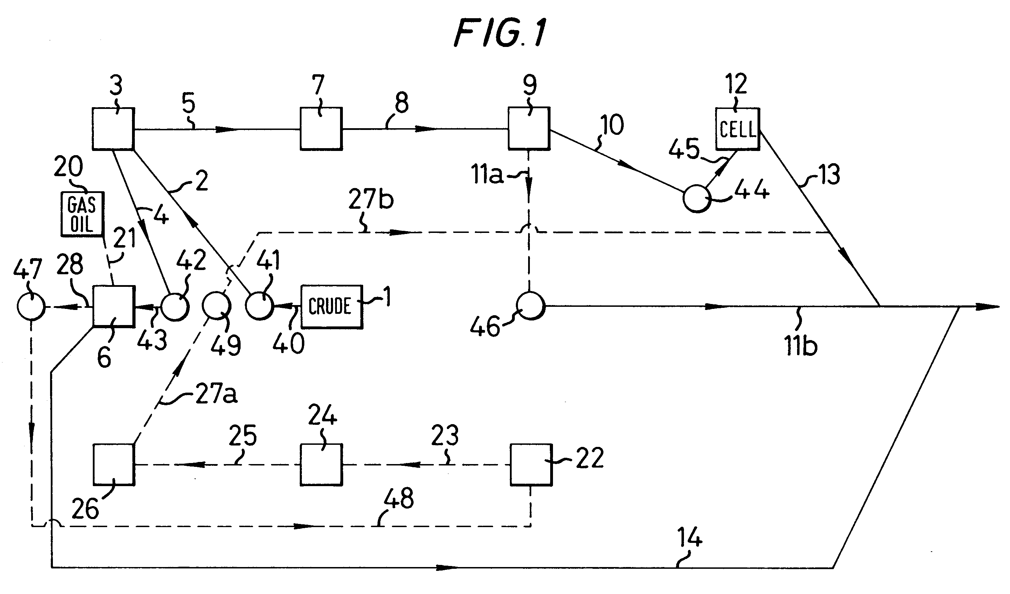

Referring to Fig. 1, a sample point in a process line 1 carrying crude oil is joined by line 40 to multi way valve 41 and thence by line 2 to a third separation means which is a filter 3 from which are two lines 4 and 5 for filtrate, line 4 for recycle of filtrate leading via multi way valve 42 and line 43 to exchanger 6 and line 5 for filtrate leading to first heat exchanger 7 which is a cooler which in turn connects via line 8 to a first separation means which is a filter or coalescer 9. Extending from the filtrate side of filter or coalescer 9 are two lines, line 10 leading to multi way valve 44 and hence by line 45 to an analysis cell 12 and effluent line 11a for recycle of filtrate. Effluent line 11a leads via multi way valve 46 to effluent line 11b. Cell 12 is in turn connected to effluent line 11b by line 13. From the exchanger 6 extends recycle line 14, which meets effluent line 11b after the latter has connected with line 13 from cell 12.

A second circuit in Fig. 1 involves entry 20 for gas oil via line 21 into heat exchanger 6, from which an oil line 28 (for hot gas oil) leads via multi way valve 47 to recycle line 48 to the filtrate side of second separation means which is a filter or coalescer22, from the top side of which leads via line 23 to second heat exchanger 24 and hence via line 25 to the filtrate (bottom) side of fifth filter 26. The top side of filter 26 is connected via line 27 to line 13 from cell 12 and hence to effluent line 14.

The filters 3 and 26 are preferably of the same porosity which may be the same as or larger than those of filters 9 and 22. The porosity of the filters may be 50-100 microns e.g. 80 microns. In use, the sample of crude oil (containing insoluble or potentially insoluble materials) is taken from the process line and passes via lines 2 and 40 and valve 4 to the third filter 3, where solid is retained and the filtrate divided, some, usually the vast majority, being recycled via lines 4 and 43 and valve 42 to third heat exchanger 6 where it is cooled and sent via line 14 to effluent. The rest of the filtrate passes via line 5 to first heat exchanger 7 where it is cooled to produce a suspension of solids which travel in line 8 to first filter or coalescer 9, where again some, but usually the vast majority, is recycled, here direct via lines 11a and 11b and valve 46 to effluent, and the rest passes as analysate via lines 10 and 45 and valve 44 to the analytical cell 12. If desired the analysate can be and preferably is brought to a constant temperature via a heat exchanger (not shown) before entering the cell; this temperature may be the same as that of filter 9 or especially may be higher. Alternatively or as well, between valve 44 and cell 12, the contents of line 45 may be heated to a temperature slightly above the temperature of filter or coalescer 9 e.g. 1-5°C above that temperature. This heating may be direct heating of the line e.g. a steam traced line or warmed regulated line, or by use of a heat exchanger in the line (now shown). The analysis cell 12 e.g. for spectroscopic analysis such as NIR is also usually kept at a constant temperature by means of a heat exchanger (now shown). The analysate leaves cell 12 and moves in line 13 to effluent. Contemporaneous with this operation, gas oil from entry 20 passes via line 21 to the back side of third exchanger 6 where it is heated prior to movement in lines 28 and 48 and valve 47 to the backside of second filter or coalescer 22, where it backwashes the filter to remove/dissolve solid residue thereon. The effluent produced, which is usually a solution in the hot oil, passes in line 23 to second heat exchanger 24 which is washed, prior to passage via line 25 to the backside of fifth filter 26, which is backwashed to produce an effluent suspension or solution which leaves the apparatus via lines 27a and 27b between which is multi way valve 49, and lines 13 and 11b.

In Fig. 1, the sampling occurs with input liquid from the process line passing to the cell via third and first filters 3 and 9, and backwash occurs with hot oil in the second line from second via fifth filters 22 and 26 to produce effluent leaving via 27.

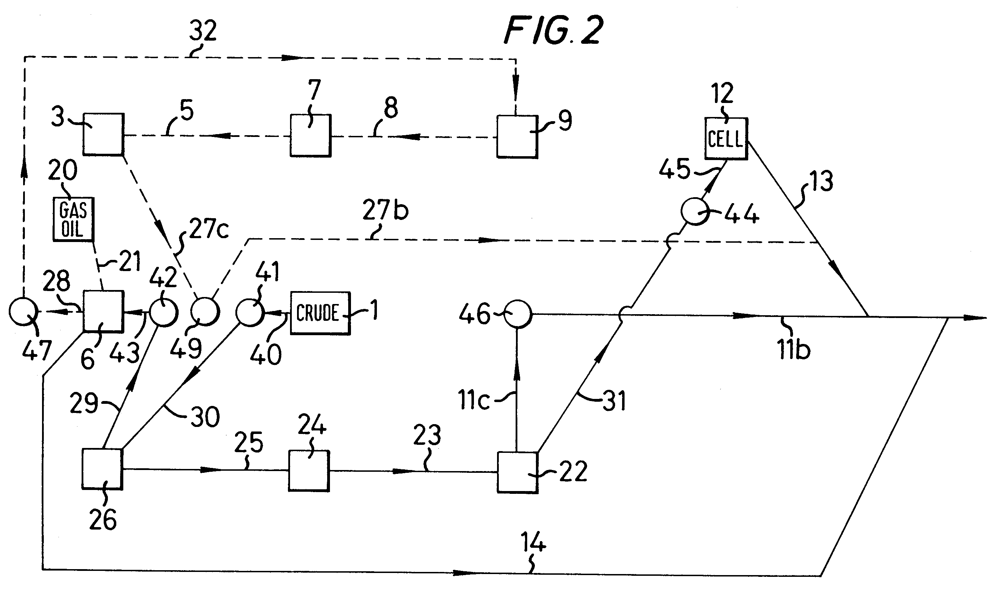

In Fig.2, the primary flows are reversed, the sampling going via fifth and second filters 26 and 22 to the cell 12, and the hot oil going via first and third filters 9 and 3 and leaving via 27. The equipment is the same as in Fig. 1, apart from a few changed lines, so only the latter will be mentioned. A line 29 joining valve 42 to fifth filter 26 replaces line 4 to third filter 3. A line 30 joining valve 41 to fifth filter 26 replaces line 2 joining it to third filter 3. Analysis line 31 joining second filter or coalescer 22 to valve 44 replaces line 10 to valve 44. A recycle line 11c from second filter or coalescer 22 to valve 46 replaces recycle line 11a from first filter or coalescer 9. A line 32 joins the backside of exchanger 6 to the backside of first filter or coalescer 9, replacing line 48 going to filter 22. A line 27c leaves the topside of third filter 3 and meets effluent line 27b at valve 49, to replace line 27a leaving fifth filter 26.

In use in Fig.2 the general operation is the opposite of that in Fig.1 so the crude oil is filtered, some recycled, the rest cooled refiltered and some recycled, while the remainder is analysed, while contemporaneously the gas oil is heated and backwashes the first and third filters prior to passing to effluent.

Periodically in order to minimize the amount of any residual deposits in the pores of the filters, at least part of the whole system, especially the first and second filters, is flushed out with a better solvent than the hot gas oil, such as carbon tetrachloride. In this case (see Fig.1) the solvent may enter at a multiway valve (not shown) in line 13, pass backwards through cell 12, line 10, and first filter 21 before leaving via line 11a; alternatively the same arrangement but in Fig.2 allows the second filter 22 to be cleaned. Under these circumstances, while the first filter is being backwashed, all the filtrate from the second filtrate is recycled, and then the reverse.

The exchangers 7 and 24 may be cooled with water, while exchanger 6 is cooled with entering gas oil.

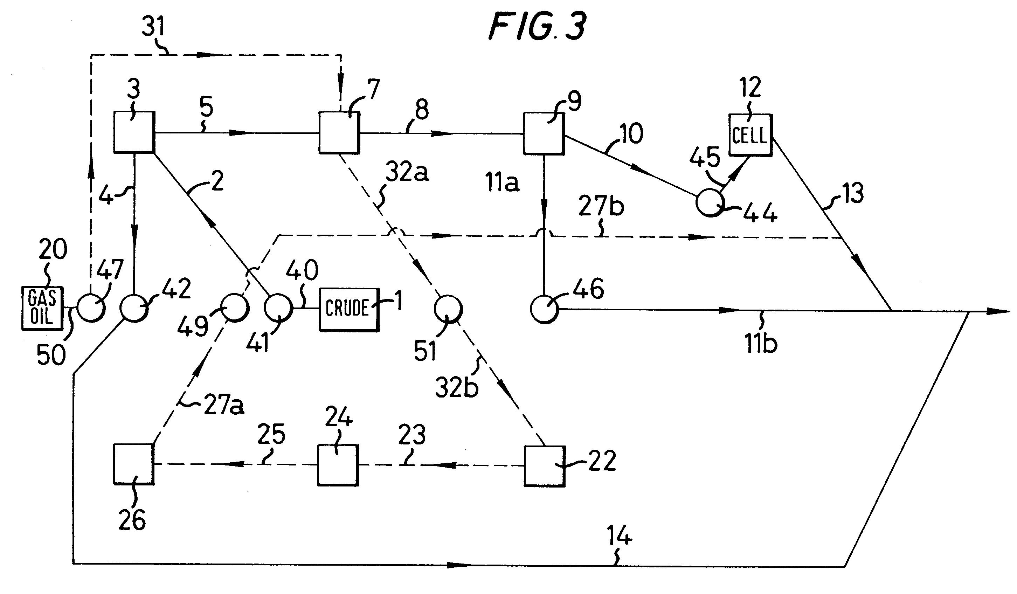

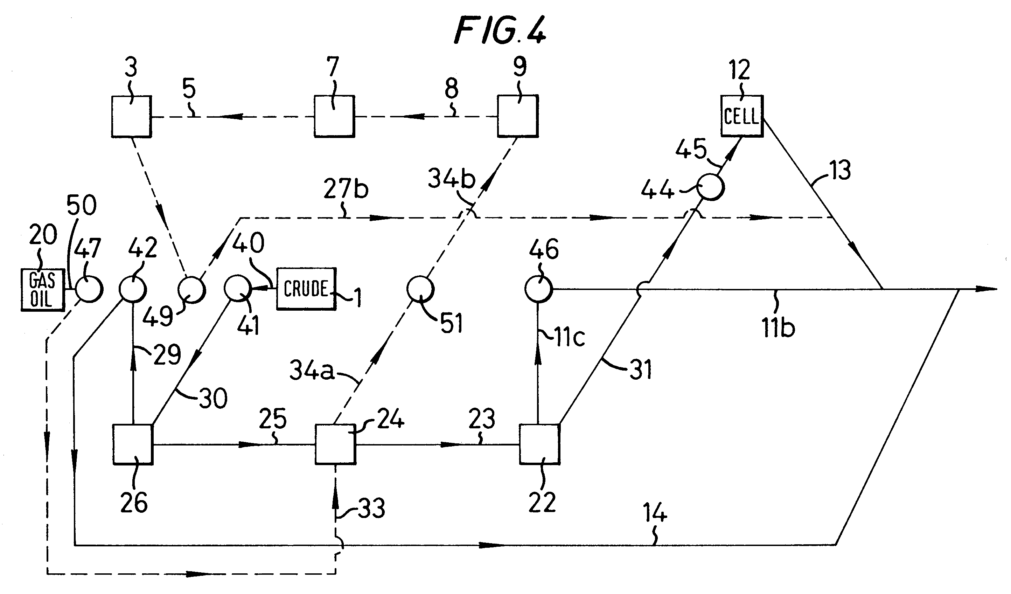

Fig.3 and 4 show the layout for another apparatus and method of the present invention. Where apparatus and flows are the same as in Figs. 1 and 2, they will not be described again, but the differences will; these particularly concern the source of the cooling fluid for coolers for the entering crude oil and the source of the heating fluid for heating the entering gas oil.

Referring to Fig. 3, the recycled filtrate from filter 3 passes in line 4, not via exchanger 6 (as in Fig.1) but direct to line 14 by way of three way valve 42. The rest of the changes concern the backwash circuit. Gas oil entry 20 is joined to multi way valve 47 by line 50 and thence by line 31 to the cooling fluid part of exchanger 7, where it cools hot crude filtrate entering in line 5. The cooled crude filtrate passes in line 8 to filter or coalescer 9 as before. The heated gas oil from exchanger 7 passes in a line 32a via multi way valve 51 and line 32b to the backside of filter or coalescer 22, which is then backwashed, the liquid from which passes in line 23 to exchanger 24 as before.

Referring to Fig.4, the recycled filtrate from filter 26 passes in line 29, not via exchanger 6 (as in Fig.2) but direct to line 14 by way of the three way valve 42. The rest of the changes concern the backwash circuit. Gas oil entry 20 is joined to multi way valve 47 via line 50 and thence by line 33 to the cooling fluid part of exchanger 24, where it cools hot crude filtrate entering in line 25. The cooled crude/filtrate passes in line 23 to filter or coalescer 22 as before. The heated gas oil from exchanger 24 passes in a line 34a via valve 51 and line 34b to the backside of filter or coalescer 9, which is then backwashed, the liquid from which passes in line 28 to exchanger 7 as before.

The filters 3, 9, 26 and 22 may be as described in relation to Fig. 1/2, or may be substantially finer e.g. 5-30 microns such as 10-20 microns.

In particular mode of use of the process of Figs.3 and 4, apparatus 9 and 22 are coalescers to remove water and the heat exchangers 7 and 24 are operated at temperatures such that water, but substantially no wax separates; this may be achieved by adjustment of the flow rate of the input gas oil in relation to the flow rate of crude oil to exchangers 7 and 24. In this case the coalescers 9 and 22 should be maintained also at the higher temperature i.e. ± 10°C of the temperature of 7 and 24 to ensure substantially no separation of wax, but only separation of water. The analysis cell 12 should also be maintained at a higher temperature to stop wax separation; this temperature is usually at or above that of 9 and 22.

If desired, not shown, there may be a further exchanger after coalescers 9 and 22 to cool the crude oil to form wax, and then a further solid filter to remove that wax before the oil reaches the cell 12.

## Claims

1. A method of on line analysis of a feed comprising a liquid and containing at least one material capable of interfering with the analytical apparatus, which method comprises the steps
(i) passing a first sample of said feed containing said material at least in part as a separate phase to a first separation means to give a first fluid and a first residue retained by said first separation means,
(ii) analysing said first fluid
(iii) removing said first residue from said first separation means
(iv) passing a second sample of said feed containing said material at least in part in a separate phase to a second separation means to give a second fluid and a second residue retained by said second separation means,
(v) analysing said second fluid
(vi) removing said second residue from said second separation means,
wherein steps (iii) and (v) are performed at least to some extent at the same time.

2. A method according to claim 1, wherein the first and second samples pass respectively through a third and fifth separation means to remove insoluble material and a first and second heat exchanger respectively to cool them before being passed to the first and second separation means respectively.

3. A method according to claim 2 wherein the third and fifth separation means remove insoluble solids, and preferably the first and second separation means remove insoluble solids or liquids.

4. A method according to any one of the preceding claims wherein the first and and second samples are crude oil containing insoluble solids and soluble waxes, which can separate on cooling before the first and second fluids reach the first and second separation means.

5. A method according to any one of the preceding claims wherein the samples are analysed by near infra red spectroscopy.

6. A method according to any one of the preceding claims wherein in steps iii and vi, a backwash liquid is passed in an opposed direction to that of the first and second samples through the first or second separation means, first or second heat exchangers, and third or fifth separation means (if present).

7. Apparatus for sampling a feed in a process line and analysing it, which apparatus provides a first and second line for attachment to said process line (preferably either directly or indirectly via a multi way valve), a first and second separation means joined to said first and second line, a first Analytical Means joined to said first separation means by a third line, and said first Analytical Means or a second Analytical Means being joined to said second separation means by a fourth line, and means for cleaning said first aid second separation means with first and second liquids.

8. Apparatus according to claim 7 wherein between the process line and first or second separation means are third or fifth separation means and first or second heat exchangers.

9. Apparatus according to claim 7 or 8 wherein a first Analytical Means comprises a near infra red spectroscopy cell and spectrophotometer.
